(19) Europäisches Patentamt
European Patent Office
Office européen des brevets

(11) Veröffentlichungsnummer : **0 467 847 A1**

(12) **EUROPÄISCHE PATENTANMELDUNG**

(21) Anmeldenummer : **91810558.6**

(51) Int. Cl.$^5$ : **C08G 65/40**

(22) Anmeldetag : **11.07.91**

(30) Priorität : **20.07.90 CH 2417/90**

(43) Veröffentlichungstag der Anmeldung :
**22.01.92 Patentblatt 92/04**

(84) Benannte Vertragsstaaten :
**DE ES FR GB IT NL SE**

(71) Anmelder : **CIBA-GEIGY AG**
**Klybeckstrasse 141**
**CH-4002 Basel (CH)**

(72) Erfinder : **Pfaendner, Rudolf, Dr.**
**Sackgasse 3**
**W-6149 Rimbach/Odenwald 1 (DE)**
Erfinder : **Wolf, Jean-Pierre, Dr.**
**257 chemin de la motta**
**CH-1791 Courtaman (CH)**
Erfinder : **Kainmüller, Thomas, Dr.**
**Am Kümmelberg 1**
**W-6145 Lindenfels 1 (DE)**
Erfinder : **Kramer, Andreas, Dr.**
**Bundtels 3**
**CH-3186 Düdingen (CH)**
Erfinder : **Hoffmann, Kurt, Dr.**
**Heidenbergstrasse 15**
**W-6147 Lautertal 2 (DE)**
Erfinder : **Stockinger, Friedrich**
**Au Fernotz**
**CH-1784 Courtepin (CH)**

(54) **Polyarylenether mit Tetraphenylethylen-Einheiten.**

(57) Polyarylenether, die, bezogen auf die Gesamtmenge der im Polymer vorhandenen Strukturelemente, 2-100 Mol% eines oder mehrerer wiederkehrender Strukturelemente der Formeln Ia, Ib oder Ic

EP 0 467 847 A1

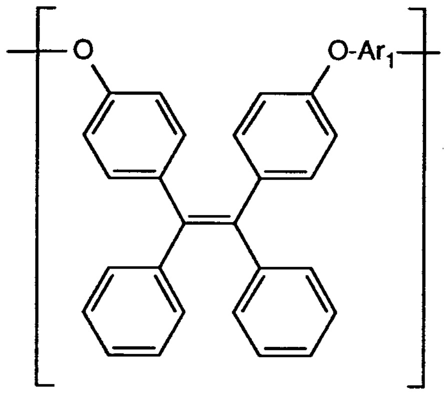

(Ia),

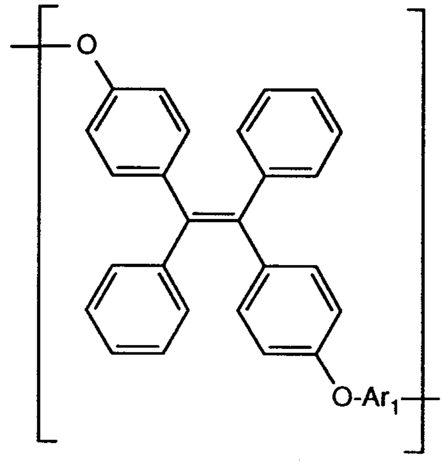

(Ib),

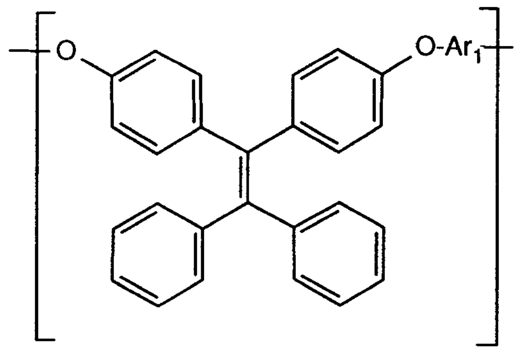

(Ic)

und 0-98 Mol% eines wiederkehrenden Strukturelementes der Formel II

$$-\!\!\left[-O-Ar_2-O-Ar_1-\right]\!\!- \qquad (II)$$

enthalten, worin $Ar_1$ und $Ar_2$ z.B. einen 4,4'-Diphenylsulfonrest bedeuten, weisen eine sehr gute Löslichkeit in einer Vielzahl von organischen Lösungsmitteln auf und lassen sich aus solchen stabilen Lösungen heraus verarbeiten.

Die Erfindung betrifft neue bestimmte Polyarylenether mit Tetraphenylethylen-Einheiten, ein Verfahren zu deren Herstellung und deren Verwendung sowie bestimmte Diphenol-Isomerengemische als Monomer-Komponente für die Synthese der Polyarylenether und ein Verfahren zur Herstellung dieser Isomerengemische.

Polyarylenether sind technische Werkstoffe mit sehr guten mechanischen und thermischen Eigenschaften, die gegenüber herkömmlichen organischen Lösungsmitteln eine hohe Beständigkeit aufweisen. Für manche Anwendungen ist jedoch diese Lösungsmittelresistenz nicht erwünscht. Insbesondere sollen bei der Modifizierung von duromeren Matrixharzen möglichst konzentrierte Polymerlösungen in den üblichen organischen Lösungsmitteln zur Verwendung kommen. Aufgabe der vorliegenden Erfindung war daher die Bereitstellung von Polyarylenethern mit sehr guter Löslichkeit in einer Vielzahl von organischen Lösungsmitteln.

Dieses Ziel wird erreicht, indem als Monomerkomponente Bis(4-hydroxyphenyl)diphenylethylen verwendet wird.

Gegenstand der Erfindung sind demnach Polyarylenether, die, bezogen auf die Gesamtmenge der im Polymer vorhandenen Strukturelemente, 2-100 Mol% eines oder mehrerer wiederkehrender Strukturelemente der Formeln Ia, Ib oder Ic

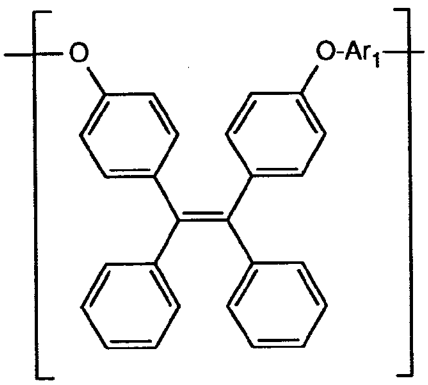
(Ia),
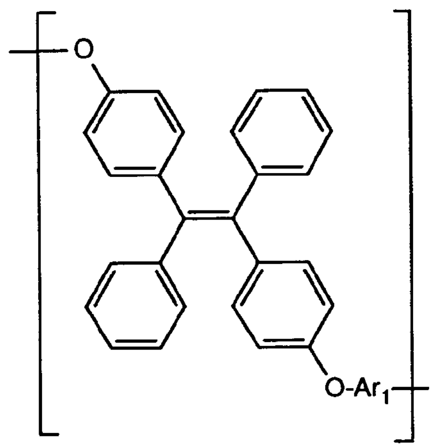
(Ib),

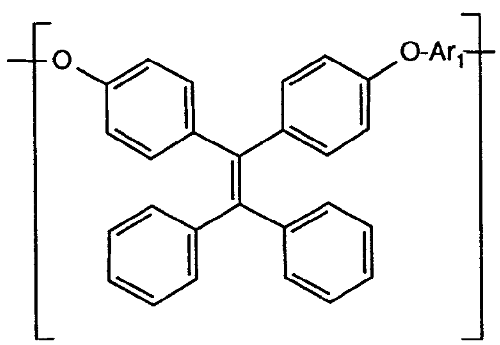
(Ic)

und 0-98 Mol% eines wiederkehrenden Strukturelementes der Formel II

$$-[-O-Ar_2-O-Ar_1-]- \qquad (II)$$

enthalten, worin $Ar_2$ einen unsubstituierten oder durch eine oder mehrere $C_1$-$C_4$-Alkyl-gruppen, $C_1$-$C_4$-Alkoxygruppen oder Halogenatome substituierten Rest der Formeln IIIa-IIIg bedeutet:

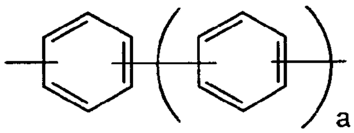 (IIIa), 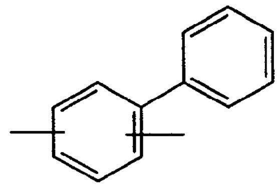 (IIIb),

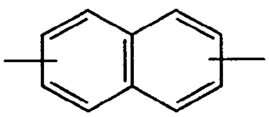 (IIIc), 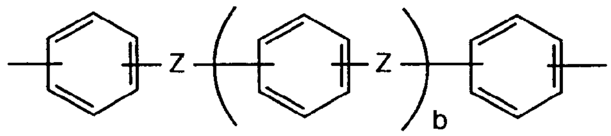 (IIId),

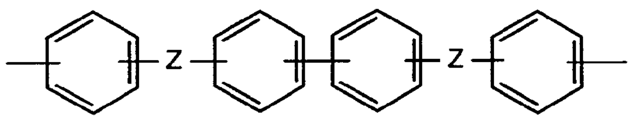 (IIIe),

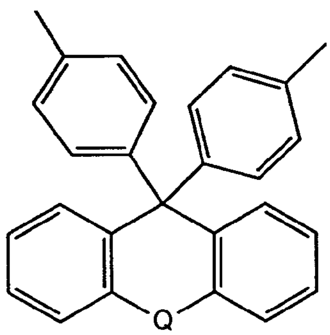 , 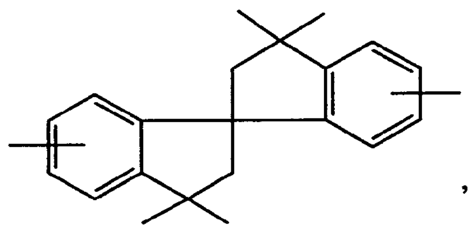 ,

(IIIf) (IIIg)

wobei a und b für Null oder 1 und Z für -CO-, $-SO_2-$, -SO-, -S-, -O-, $-C(CH_3)_2-$, $-C(CF_3)_2-$, $-CH_2-$ oder $-C(CH_3)(C_6H_5)-$ stehen und Q für $-CH_2-$, -O-, -C(=O)- oder eine direkte Bindung steht, und $Ar_1$ einen unsubstituierten oder durch eine oder mehrere $C_1$-$C_4$-Alkylgruppen, $C_1$-$C_4$-Alkoxygruppen oder Halogenatome substituierten Rest der Formeln IVa, IVb oder IVc bedeutet

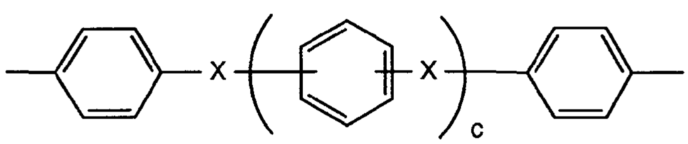 (IVa),

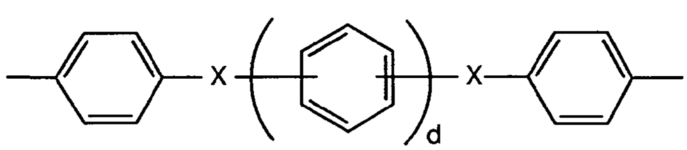 (IVb),

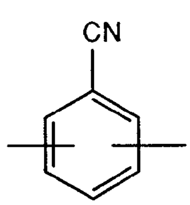 (IVc),

wobei c für Null, 1 oder 2 und d für 2 oder 3 stehen und X -CO-, $-SO_2-$ oder -SO- darstellt.

Polyarylenether, die eine Tetraphenylethylen-Struktur beinhalten, sind bisher in der Literatur nicht bekannt.

Als einzige Polymere, die eine Tetraphenylethylen-Einheit aufweisen, sind Polyamide und Polyimide bekannt (Y. Oishi et al. Pol.Mat.Sci.Eng. 1989, 60, 757). Die dort beschriebenen Polymeren werden ausgehend von 4,4′-Diaminotetraphenylethylen synthetisiert, sie sind je nach Reaktionspartner in aprotischen, dipolaren Lösungsmitteln, wie N-Methylpyrrolidon, Dimethylsulfoxid, Dimethylacetamid oder Pyridin, löslich. Solche Löslichkeitseigenschaften sind aber für Polyamide bzw. Polyimide keinesfalls hervorragend, da sich andere bekannte Polymere, wie z.B. die von Diaminotrimethylphenylindan abgeleiteten Polyimide gemäss dem US-Patent Nr. 3,856,752 und der EP-Patentanmeldung Nr. 92,524, durch wesentlich bessere Löslichkeiten auszeichnen. Weitere Polyamide mit Tetraphenylethylen-Einheiten sind auch in J. of Polymer Science, Part A, 1991, Vol. 29, 55-61 beschrieben.

Es war daher überraschend, dass die erfindungsgemässen Polyarylenether auf der Basis von Tetraphenylethylen-Einheiten besonders gute Löslichkeiten in üblichen organischen Lösungsmitteln, wie chlorierte Kohlenwasserstoffe, cyclische Ketone oder cyclische Ether, aufweisen.

Andere bekannte Polyarylenether, deren Monomerkomponente ebenfalls vier Phenylgruppen aufweist, wie z.B. die von 4,4′-Dihydroxy-3,3′-diphenyl-biphenyl abgeleiteten Polyarylenether gemäss der DE-Patentanmeldung Nr. 3,825, 148, haben nicht die ausgezeichneten Löslichkeitseigenschaften der vorliegenden Verbindungen.

Die erfindungsgemässen Polyarylenether können so hergestellt werden, dass man ein oder mehrere Diphenole der Formeln Va, Vb oder Vc

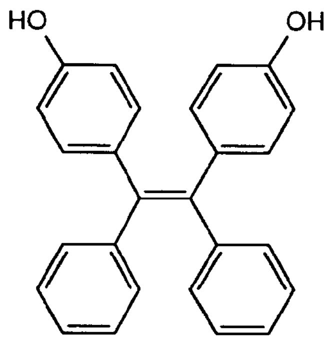 (Va), 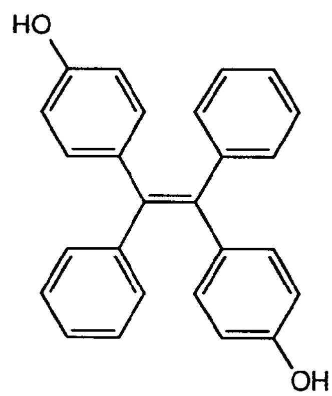 (Vb),

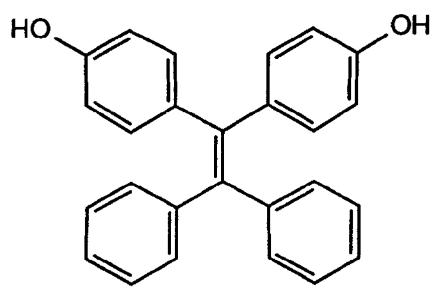 (Vc)

und gegebenenfalls ein Diphenol der Formel VI

$$HO\text{-}Ar_2\text{-}OH \quad (VI)$$

mit einem Dihalogenderivat der Formel VII

$$Hal\text{-}Ar_1\text{-}Hal \quad (VII)$$

in Gegenwart alkalischer Katalysatoren in einem polaren aprotischen Lösungsmittel polykondensiert, wobei $Ar_1$ und $Ar_2$ die oben angegebene Bedeutung haben, und Hal für Halogen, insbesondere Fluor oder Chlor, steht, und wobei die relativen Mengen des Diphenols gemäss Formeln Va bis Vc und des Diphenols der Formel VI so gewählt werden, dass das resultierende Copolymer die im Anspruch 1 definierten Mengen der Strukturelemente der Formel I und II aufweist.

Anstelle der Diphenole der Formeln V oder VI können auch die entsprechenden Alkali-oder Erdalkaliphenolate, z.B. die Kalium- oder Calciumphenolate, eingesetzt werden.

Ueblicherweise wird die Polykondensation in etwa äquimolaren Verhältnissen der Diphenole der Formeln

V und gegebenenfalls VI, bezogen auf das Dihalogenderivat der Formel VII, durchgefürt. Unter etwa äquimolaren Mengen versteht man ein Molverhältnis von 0,8:1,0 bis 1,0:0,8; Bevorzugt ist ein Molverhältnis von 0,95 : 1,00 bis 1,00 : 0,95.

Bei der Herstellung der Copolymeren, welche sowohl Strukturelemente der Formel I als auch Strukturelemente der Formel II enthalten, kann die Herstellung sowohl in statistischer als auch in segmentierter Weise (Blockcopolymeres) erfolgen. Bei der Synthese in statistischer Art wird z.B. ein Gemisch der Phenole V und VI mit einem oder mehreren Dihalogenderivaten der Formel VII kondensiert.

Zur Herstellung eines Blockcopolymeren werden Reaktionsprodukte der Umsetzung eines Diphenols der Formel V und eines Dihalogenderivats der Formel VII mit Hydroxyl- oder Phenolat-Endgruppen und die Reaktionsprodukte der Umsetzung eines Diphenols der Formel VI und eines Dihalogenderivats der Formel VII mit Halogenendgruppen oder umgekehrt synthetisiert. Dies wird bekannterweise durch Einsatz eines geeigneten Ueberschusses des Diphenols bzw. des Dihalogenderivates bei der Herstellung des Reaktionsproduktes erreicht. Ein geeigneter Ueberschuss eines der Edukte ist beispielsweise ein Ueberschuss von etwa 1 bis 50 Mol.%. Die Höhe des Ueberschusses bestimmt jeweils die Blocklänge.

Als alkalische Katalysatoren verwendet man im erfindungsgemässen Verfahren in der Regel Alkalimetall- und Erdalimetallcarbonate sowie die entsprechenden Hydrogencarbonate, wie Natrium-, Kalium- oder Calciumcarbonat oder -hydrogencarbonat; doch können auch andere alkalische Reagentien, wie Natriumhydroxid, Kaliumhydroxid oder Calciumhydroxid, eingesetzt werden.

Polare, aprotische Lösungsmittel, die zur Herstellung der erfindungsgemässen Polyarylenetherharze eingesetzt werden können, sind beispielsweise Dimethylsulfoxid, Dimethylacetamid, Diethylacetamid, Tetramethylharnstoff, N-Methylcaprolactam, N-Methylpyrrolidon und bevorzugt Diphenylsulfon.

Die Reaktion wird zweckmässig bei erhöhter Temperatur durchgeführt, vorzugsweise bis zur Rückfluss-Temperatur des Lösungsmittels, z.B. etwa bis 350°C.

Häufig empfiehlt es sich, ein Schleppmittel, wie z.B. Chlorbenzol, Xylol oder Toluol, mitzuverwenden, um das bei der Umsetzung gebildete Wasser aus dem Reaktionsgemisch azeotrop entfemen zu können.

Die Verbindungen der Formel VII sind bekannt. Sie sind zum Teil im Handel erhältlich oder können auf bekannte Art und Weise hergestellt werden.

Geeignete Verbindungen der Formel VII sind beispielsweise 4,4′-Dichlor- oder 4,4′-Difluordiphenylsulfoxid oder -diphenylsulfon, 4,4′-Dichlor- oder 4,4′-Difluorbenzophenon, 4,4′-Dichlorisophthalophenon und 4,4′-Dichlorterephthalophenon, oder 2,6-Difluorbenzonitril oder 2,6-Dichlorbenzonitril.

Bevorzugte Verbindungen der Formel VII sind 4,4′-Difluorbenzophenon und insbesondere 4,4′-Dichlordiphenylsulfon.

Verbindungen der Formel VI sind ebenfalls bekannt und sind im Handel erhältlich. Geeignete Verbindungen der Formel VI sind z.B. Hydrochinon, 4,4′-Dihydroxybiphenyl, 2-Phenylhydrochinon, 2,6- und 2,7-Dihydroxynaphthalin, Bisphenol A, Bisphenol F, 4,4′-Dihydroxybenzophenon, 4,4′-Dihydroxydiphenylether, 4,4′-Dihydroxydiphenylsulfid, 4,4′-Dihydroxydiphenylsulfoxid, 4,4′-Dihydroxydiphenylsulfon oder 2,2-Bis(4′-hydroxyphenyl)-1,1,1,3,3,3-hexafluorpropan, sowie die entsprechenden $C_1$-$C_4$-alkylsubstituierten Derivate, wie z.B. 3,3′,5,5′-Tetramethyl-4,4′-dihydroxydiphenylsulfon.

Weitere geeignete Verbindungen sind: 2,2′-Dihydroxybiphenyl, 9,9-Bis(4′-hydroxyphenyl)fluoren und 6,6′-Dihydroxy-3,3,3′,3′-tetramethyl- 1,1′-spirobiindan.

Bevorzugte Verbindungen der Formel VI sind 4,4′-Dihydroxybiphenyl, 4,4′-Dihydroxybenzophenon, Hydrochinon und Bisphenol A.

Eine besonders bevorzugte Verbindung der Formel VI ist 4,4′-Dihydroxydiphenylsulfon.

Die Diphenole der Formeln Va, Vb und Vc sind bekannt bzw. können auf bekannte Weise hergestellt werden. Ein geeigneter Syntheseweg für die Herstellung von 1,2-Bis(4′-hydroxyphenyl)-1,2-diphenylethylen ist von R.N. Iyer und K.V.B. Rao in Ind.J.Chem., 16B, 601 (1978) beschrieben. Dabei wird zuerst 4-Methoxybenzophenon mit $PCl_5$ zum 4-Methoxyphenyl-phenyldichlormethan übergeführt, das resultierende Dichlormethanderivat wird durch Erhitzen in einem Lösungsmittel in Gegenwart von elementarem Kupfer zum tetrasubstituierten Ethylenderivat dimerisiert und anschliessend wird der Methylether durch Erhitzen mit Kaliumhydroxid in Diethylenglykol gespalten. Eine analoge Synthese von Tetraphenylethylenderivaten ausgehend von unsubstituiertem Benzophenon ist in Org.Synth.Coll.Vol. II, 573 (1943) und Coll.Vol. IV, 914 (1963) beschrieben.

Es hat sich gezeigt, dass besonders gut lösliche Polyarylenether erhalten werden, wenn bei deren Herstellung als Diphenolkomponente ein Dihydroxytetraphenylisomerengemisch enthaltend die Diphenole der Formeln Va, Vb und Vc verwendet wird. Dieses Isomerengemisch ist neu und ist ebenfalls Gegenstand vorliegender Erfindung. Besonders bevorzugt ist ein Isomerengemisch enthaltend die Diphenole Va, Vb und Vc in einem Molverhältnis von etwa 2:2: 1. Das erfindungsgemässe Isomerengemisch ist z.B. durch Spaltung des oben beschriebenen Tetraphenylethylendimethylethers in stark saurem Medium, beispielsweise mit HBr in Eisessig, erhältlich währenddem die in Ind. J. Chem. 1978, 16 B, 601 beschiebene entsprechende alkalische

Spaltung ein Gemisch enthaltend nur zwei Isomere liefert. Dieses Herstellungsverfarhen für das Isomerengemisch ist ebenfalls Gegenstand vorliegender Erfindung.

Vorzugsweise enthalten die erfindungsgemässen Polyarylenether 15-100, insbesondere 10-100 Mol% eines wiederkehrenden Strukturelements der Formel I und 0-95, insbesondere 0-90 Mol% eines wiederkehrenden Strukturelements der Formel II; Besonders bevorzugt dabei bedeuten $Ar_1$ und $Ar_2$ in den Formeln I bzw. II einen Rest der Formel

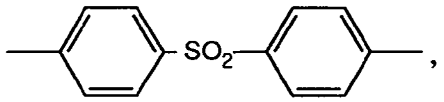

oder $Ar_1$ einen Rest der Formel

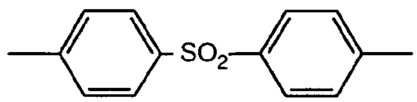

und $Ar_2$ einen Rest der Formel

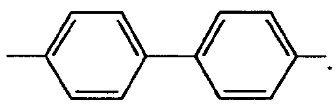

Besonders bevorzugte Polyarylenether enthalten 25- 100 Mol% eines wiederkehrenden Strukturelements der Formel I und 0-75 Mol% eines wiederkehrenden Strukturelementes der Formel II.

In den Formeln IVa und IVb bedeutet X bevorzugt $-SO_2-$. Die Gruppe $Ar_1$ steht vorzugsweise für den Rest

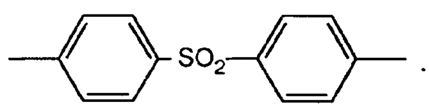

Die bevorzugte Bedeutung von Z in den Formeln IIId und IIIe ist $-C(CH_3)_2-$, -CO-, $-SO_2-$, -S- oder -O-.

Die Phenylengruppen in den Formeln IIIa bis IIIe und IVa und IVb sind vorzugsweise 1,3- und insbesondere 1,4-Phenylengruppen. Die Alkyl- oder Alkoxysubstituenten der Gruppen $Ar_1$ und $Ar_2$ können geradkettig oder verzweigt sein. Beispiele für geeignete Substituenten sind Ethyl, n-Propyl, Isopropyl, n-Butyl und insbesondere Methyl, sowie die entsprechenden Alkoxygruppen. Bevorzugte Gruppen $Ar_1$ und $Ar_2$ sind unsubstituiert.

Die Gruppe $Ar_2$ der erfindungsgemässen Verbindungen steht vorzugsweise für einen Rest der Formeln

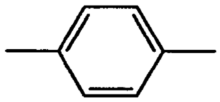 , 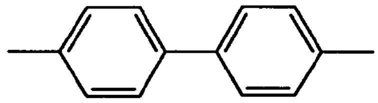 , 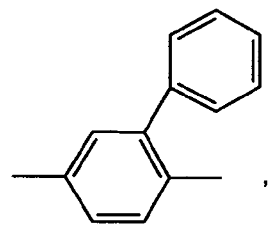 ,

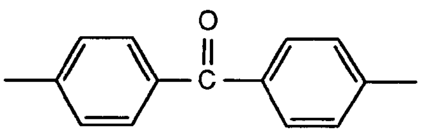 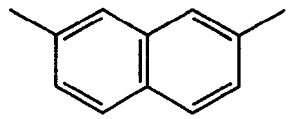 ,

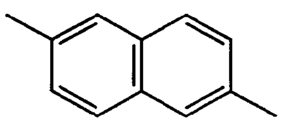 , 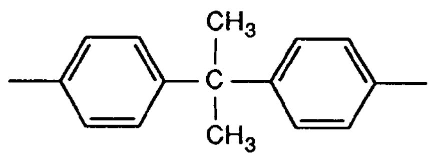 ,

oder insbesondere

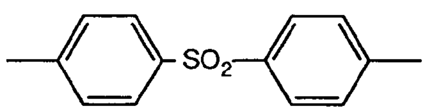 .

Die erfindungsgemässen Polyarylenether weisen vorzugsweise eine reduzierte Viskosität von 0,1 bis 2,0 dl/g, insbesondere von 0,3 bis 1,5 dl/g, gemessen bei 25°C an einer 1 %igen Lösung in N-Methylpyrrolidon (1 g Polymer in 100 ml NMP gelöst), auf.

Die erfindungsgemässen Polyarylenether können auf für Thermoplaste übliche Art und Weise eingesetzt und z.B. zu Formkörpem oder Folien verarbeitet werden, oder als Matrixharze, Klebstoffe oder Ueberzugsmittel eingesetzt werden. Vor der Verarbeitung der beispielsweise als Presspulver, Schmelze oder Lösung vorliegenden Polyarylenether können übliche Zusatzstoffe, wie beispielsweise Füllstoffe, Pigmente, Stabilisatoren oder Verstärkungsmittel, wie kohlenstoff-, Bor- oder Glasfasern, zugegeben werden. Die erfindungsgemässen Polyarylenether können auch zusammen mit anderen Thermoplasten verarbeitet werden.

Vorzugsweise eignen sich die erfindungsgemässen Polyarylenether als Matrixharze für die Herstellung von Faserverbundsystemen, wobei man als Verstärkungsfasern die üblichen bei der Verstärkung technischer Werkstoffe verwendeten Fasern einsetzen kann. Diese können organische oder anorganische Fasern, Naturfasern oder Synthesefasern, wie Aramid-Fasern, sein, und als Faserbündel, als orientierte oder nicht-orientierte Fasern oder als Endlosfasern vorliegen. Als Verstärkungsfasern verwendet man beispielsweise Glas-, Bor-, Kohlenstoff- und Metallfasern.

Eine weitere bevorzugte Anwendungsmöglichkeit für die erfindungsgemässen Polyarylenether besteht in der Modifizierung anderer Kunststoffe. Diese Kunststoffe können grundsätzlich Thermoplaste oder Duromere sein. Besondere Bedeutung erlangte die Modifizierung von hitzehärtbaren Harzen, insbesondere von Epoxidharzen oder Bismaleinimiden. Solchen besonderen Bedürfnissen angepassten Polymersysteme sind beispielsweise im US-Patent Nr. 3,530,087 beschrieben. Besondere Bedeutung erlangten derartige Systeme als Matrixharze zur Herstellung von Verbundbauteilen. Ueblicherweise werden etwa 5-100, vorzugsweise 10-50 Gew.-Teile Polyarylenether pro 100 Gew.-Teile zu modifizierender Kunststoffe eingesetzt.

Gegenstand vorliegender Erfindung sind somit auch Formkörper, Folien, Beschichtungen oder Klebeverbindungen enthaltend einen erfindungsgemässen Polyarylenether sowie Faserverbunde enthaltend Verstärkungsfasem und als Matrixharz einen erfindungsgemässen Polyarylenether.

Besonders hervorzuheben sind sowohl die sehr gute Löslichkeit der erfindungsgemässen Polymeren in einer Vielzahl von organischen Lösungsmitteln (z.B. in chlorierten Kohlenwasserstoffen, cyclischen Ketonen, cyclischen Ethern) als auch die sehr gute Stabilität dieser Lösungen. Die Polymeren lassen sich somit aus einer Lösung heraus verarbeiten, z.B. zu Filmen, oder in andere Systeme, z.B. Matrixharze, einbringen. Lösungen enthaltend 1-75 Gew.%, vorzugsweise 5-40 Gew.%, der erfindungsgemässen Polyarylenether in einem organischen Lösungsmittel stellen somit einen weiteren Erfindungsgegenstand dar. Bevorzugte organische Lösungsmittel sind z.B. N-Methylpyrrolidon, γ-Butyrolacton, Dimethylacetamid, Dimethylsulfoxid, Cyclohexanon, Cyclopentanon, 1,3-Dioxolan und Methylenchlorid. Bevorzugte Lösungen enthalten einen Polyarylenether, der 5- 100, vorzugsweise 10- 100 Mol% eines wiederkehrenden Strukturelementes der Formel I und 0-95, vorzugsweise 0-90 Mol% eines wiederkehrenden Strukturelementes der Formel II.

Die folgenden Beispiele erläutern die Erfindung.

Beispiel 1 : Synthese von Bis(4-hydroxyphenyl)-diphenylethylen (Isomerengemsich)

a) Bis(4-methoxyphenyl)-diphenylethylen

Eine Mischung von 212,2 g (1 mol) 4-Methoxybenzophenon und 250 g $PCl_5$ in 1,51 trockenem Benzol wird 16 Stunden am Rückfluss gekocht. Anschliessend wird das Lösungsmittel am Rotationsverdampfer abdestilliert, der ölige Rückstand wird noch dreimal mit je 300 ml Benzol versetzt, und das Lösungsmittel wird jeweils am Rotationsverdampfer abdestilliert. Der verbleibende ölige Rückstand wird mit 1,2l Benzol und 427 g Kupferpulver versetzt und 24 Stunden am Rückfluss gekocht. Die heisse Reaktionslösung wird filtriert, und das Filtrat wird am Rotationsverdampfer eingeengt. Der Rückstand wird dann aus 650 ml Eisessig umkristallisiert, die Mutterlauge wird eingedampft, und der Rückstand wird über Silicagel chromatographiert (Hexan/Toluol 3:2).

Die aus der Kristallisation aus Eisessig und der Chromatographie gewonnene Menge Bis(4-methoxyphenyl)-diphenylethylen beträgt 156 g (79,4 %).

| Elemetaranalyse: | | |
|---|---|---|
| Berechnet für $C_{28}H_{24}O_2$: | C: 85,68 | H: 6,16 |
| Gefunden: | C: 85,02 | H: 6,10 |

b) Bis(4-hydroxyphenyl)-diphenylethylen (Isomerengemisch)

Eine Suspension von 96,6 g (0,246 mol) Bis-(4-methoxyphenyl)-diphenylethylen in 486 ml Eisessig und 486 ml 48 % HBr wird 24 Stunden am Rückfluss gekocht. Die auf Raumtemperatur abgekühlte Reaktionslösung wird auf 21 Wasser gegossen, und der Niederschlag wird abfiltriert. Der getrocknete Niederschlag wird über Silicagel chromatographiert (Hexan/EtOAc 2:1).

Ausbeute an Bis(4-hydroxyphenyl)-diphenylethylen (Isomerengemisch): 64,5 g (72 %).

| Elemetaranalyse: | | |
|---|---|---|
| Berechnet für $C_{26}H_{20}O_2$: | C: 85,69 | H: 5,53 |
| Gefunden: | C: 85,52 | H: 5,54 |

Titration der Phenolgruppen: Titration in Pyridin mit 0,1 N Tetrabutylammoniumhydroxid in Isopropanol: 5,46 mAeq./g (99.4 % der Theorie).

HPLC:

Nucleosil C-18 5 μm, $CH_3CN$: $H_2O$ 7:3 (1 ml/min);
Detektion bei 254 nm;
Isomerengemisch, bestehend aus 3 Isomeren im Verhältnis 2:2:1.

Beispiel 2: Polyethersulfon aus Bis(4-hydroxyphenyl)-diphenylethylen (Isomerengemisch) und 4,4′-Dichlordiphenylsulfon

In einem Rundkolben mit Rührer und Schutzgasanschluss wird unter Stickstoff eine Mischung aus 18,34 g (0,0503 mol) Bis(4-hydroxyphenyl)-diphenylethylen (Isomerengemisch), 59,31 g Diphenylsulfon, 7,30 g (0,0528 mol) Kaliumcarbonat und 52 g Xylol bei einer Badtemperatur von 200°C erhitzt, und ein Xylol/Wasser-Gemisch wird abdestilliert. Gegen Ende des Destillationsvorganges wird kurzeitig Vakuum (2 mbar) angelegt. Sodann werden 15,38 g (0,0501 mol) 4,4′-Dichlordiphenylsulfon zu der Reaktionsmischung gegeben, die Temperatur wird innerhalb von 15 Minuten auf 228°C erhöht und 1 Stunde so belassen. Danach wird die Temperatur auf 250°C (1 Stunde) und anschliessend auf 280°C erhöht. Diese Temperatur wird 4 Stunden beibehalten. Bei dieser Temperatur werden dann 0,040 g (0,0014 mol) 4,4'-Dichlordiphenylsulfon zugegeben. Dieser Vorgang wird nach 30 Minuten und noch einmal nach 15 Minuten wiederholt. Nach weiteren 30 Minuten wird die viskose Reaktionsmischung nach kurzem Abkühlen dem Reaktionskolben entnommen.

Das erstarrte Reaktionsgemisch wird pulverisiert, dann mit Essigsäure versetzt und zunächst mit Wasser extrahiert. Anschliessend wird das Polymer in Methylenchlorid gelöst, eine geringe Menge unlösliches Material wird abfiltriert, und das gelöste Produkt wird dann mit Isopropanol ausgefällt. Das so gereinigte Polymere wird dann im Vakuumtrockenschrank bei 240°C getrocknet. Ein auf diese Weise hergestelltes Polyarylenethersulfon besitzt eine reduzierte Viskosität [1 Gew.-% Polymeres in N-Methylpyrrolidon (NMP) bei 25°C] von 0,29 dl/g. Die durch DSC bestimmte Glasübergangstemperatur liegt bei 215°C. Das $^{13}C$-NMR-Spektrum des Polyethersulfons zeigt zusätzlich zur Diphenylsulfon-Einheit dem Monomeren (Beispiel 1b) vergleichbare Signale. Insbesondere kann das unveränderte Vorhandensein der C-C-Doppelbindung nachgewiesen werden. Die besonders gute Löslichkeit dieses Polymeren ist aus der folgenden Tabelle ersichtlich.

| Lösungsmittel | NMP | Dioxan | Methylenchlorid |
|---|---|---|---|
| Beispiel 1 | + | + | + |

+ = löslich

Beispiel 3: Polyethersulfon aus Bis(4-hydroxyphenyl)-diphenylethylen (Isomerengemisch), 4,4'-Dihydroxydiphenylsulfon (1:1) und 4,4'-Dichlordiphenylsulfon

Analog dem Beispiel 1 wird ein Polymeres aus 0,0501 mol Bis(4-hydroxyphenyl)-diphenylethylen, 0,0501 mol 4,4'-Dihydroxydiphenylsulfon, 0,1000 mol 4,4'-Dichlordiphenylsulfon und 0, 1052 mol Kaliumcarbonat hergestellt (Reaktionsbedingungen: 1 h/250°C, 5 h/280°C). Das resultierende Polymere weist eine reduzierte Viskosität von 0,42 dl/g und eine Glasübergangstemperatur von 203°C auf und ist in Tetrahydrofuran, in Dioxan, in Cyclohexanon, in Cyclopentanon und in Methylenchlorid löslich.

Beispiel 4: Polyethersulfon aus Bis(4-hydroxyphenyl)-diphenylethylen (Isomerengemisch), 4,4'-Dihydroxydiphenylsulfon (1:3) und 4,4'-Dichlordiphenylsulfon

Wie in Beispiel 1 wird ein Polymeres aus 0,0504 mol Bis(4-hydroxyphenyl)-diphenylethylen, 0,1510 mol 4,4'-Dihydroxydiphenylsulfon, 0,2003 mol 4,4'-Dichlordiphenylsulfon und 0,2104 mol Kaliumcarbonat hergestellt (Reaktionsbedingungen: 1 h/250°C, 5 h/280°C). Das resultierende Polymere hat eine reduzierte Viskosität von 0,46 dl/g und eine Glasübergangstemperatur von 221°C und ist in Methylenchlorid in mehr als 25 % löslich.

Beispiel 5: Polyethersulfon aus Bis(4-hydroxyphenyl)-diphenylethylen (Isomerengemisch), 4,4'-Dihydroxydiphenylsulfon (1:9) und 4,4'-Dichlordiphenylsulfon

Wie in Beispiel 1 wird ein Polymeres aus 0,0402 mol Bis(4-hydroxyphenyl)-diphenylethylen, 0,3610 mol 4,4'-Dihydroxydiphenylsulfon, 0,4000 mol 4,4'-Dichlordiphenylsulfon und 0,420 mol Kaliumcarbonat hergestellt (Reaktionsbedingungen: 1 h/250°C, 1 h/275°C, 7 h 10 min/280°C). Das resultierende Polymere hat eine reduzierte Viskosität von 0,48 dl/g und eine Glasübergangstemperatur von 223°C und ist in Methylenchlorid zu mehr als 25 % löslich.

Beispiel 6: Polyethersulfon aus Bis(4-hydroxyphenyl)-diphenylethylen (Isomerengemisch), 4,4'-Dihydroxydiphenylsulfon (1:19) und 4,4'-Dichlordiphenylsulfon

In analoger Weise wie in Beispiel 1 wird ein Polymer aus 0,0201 mol Bis(4-hydroxyphenyl)-diphenylethylen, 0,3811 mol 4,4'-Dihydroxydiphenylsulfon, 0,4000 mol 4,4'-Dichlordiphenylsulfon und 0,420 mol Kaliumcarbonat (Reaktionsbedingungen: 1 h/250°C, 1 h/275°C, 4 h/280°C) hergestellt. Das resultierende Polymer mit einer reduzierten Viskosität von 0,49 dl/g und einer Glasübergangstemperatur von 222°C ist in Methylenchlorid zu

mehr als 25 % löslich.

Anwendungsbeispiel 7 (Polyethersulfon/Epoxy-Blend):

Ein nach Beispiel 5 hergestelltes Polyethersulfoncopolymers wird zu 20 bzw. 30 Gewichtsteilen als Lösung in Methylenchlorid zu einer Mischung, bestehend aus 50 Teilen Tetraglycidyldiaminodiphenylmethan und 50 Teilen Triglycidyl-p-aminophenol, gegeben, und das Lösungsmittel wird im Vakuum entfernt. Nach Zugabe von 50 Teilen p-Diaminodiphenylsulfon wird das Gemisch in einer Form 2 Stunden bei 160°C sowie 2 Stunden bei 210°C ausgehärtet. Aus einer so hergestellten Platte werden Prüfkörper geschnitten, und die Biegefestigkeit und Randfaserdehnung werden nach ISO 178 sowie die Bruchzähigkeit ($G_{Ic}$, mittels "bend notch" gemäss ASTM E 399) bestimmt.

Es ergeben sich folgende sehr gute Werte:

| | Teile 20 | Thermoplast 30 |
|---|---|---|
| Biegefestigkeit [N/mm$^2$]: | 166 | 170 |
| Randfaserdehnung [% ]: | 6,7 | 6,5 |
| Bruchzähigkeit [J/m$^2$]: | 171 | 300 |

Beispiel 8:

Polyetherketon aus Bis- (4-hydroxyphenyl)-diphenylethylen (Isomerengemisch) und 4,4'-Difluorbenzophenon.
In analoger Weise wie in Beispiel 1 wird ein Polymer aus 0,0100 mol Bis (4-hydroxyphenyl)-diphenylethylen, 0,0100 mol 4,4'-Difluorbenzophenon und 0,0109 mol Kaliumcarbonat (Reaktionsbedingungen: 1h/226°C, 1h/250°C und 4h/278 bis 280°C) synthetisiert. Das gemahlene Reaktionsgemisch wird mit essigsaurem Wasser, Wasser und anschliessend mit einem Wasser/Aceton (2/1) Gemisch extrahiert, in Methylenchlorid gelöst, filtriert, in Isopropanol gefällt und im Vakuumtrockenschrank bis 200°C getrocknet. Das resultierende Polyetherketon mit einer reduzierten Viskosität von 0,39 dl/g und einer Glasübergangstemperatur von 190°C ist in Methylenchlorid und N-Methylpyrrolidon zu mehr als 20 Gew.% löslich.

Beispiel 9:

Polyethersulfon aus Bis(4-hydroxyphenyl)-diphenylethylen (Isomerengemisch), 4,4'-Dihydroxybiphenyl und 4,4'-Dichlordiphenylsulfon.
In analoger Weise wie in Beispiel 8 wird ein Polymer aus 0,0251 mol Bis(4-hydroxyphenyl)-diphenylethylen, 0,0251 mol 4,4'-Dihydroxybiphenyl und 0,0500 mol 4,4'-Dichlordiphenylsulfon und 0,0526 mol Kaliumcarbonat (Reaktionsbedingungen: 1h/224°, 1h/250°C und 4h/275 bis 280°C) synthetisiert und isoliert. Das resultierende Polyethersulfon mit einer reduzierten Viskosität von 0,23 dl/g und einer Glasübergangstemperatur von 191°C ist in Methylenchlorid, in 1,3-Dioxolan, in Cyclohexanon und in N-Methylpyrrolidon zu mehr als 20 Gew.% löslich.

Beispiel 10:

Polyethersulfon aus Bis(4-hydroxyphenyl)-diphenylethylen (Isomerengemisch), Phenylhydrochinon und 4,4'-Dichlordiphenylsulfon.
In analoger Weise wie in Beispiel 8 wird ein Polymer aus 0,0251 mol Bis(4-hydroxyphenyl)-diphenylethylen, 0,0251 mol Phenylhydrochinon und 0,0500 mol 4,4'-Dichlordiphenylsulfon und 0,0525 mol Kaliumcarbonat (Reaktionsbedingungen: 1 h/226°C, 1 h/251°C und 4h/281°C) synthetisiert und isoliert. Das resultierende Polyethersulfon mit einer reduzierten Viskosität von 0,36 dl/g und einer Glasübergangstemperatur von 198°C ist in Methylenchlorid, in 1,3-Dioxolan, in Cyclohexanon und in N-Methylpyrrolidon zu mehr als 20 Gew.% löslich.

Beispiel 11:

Polyethersulfon aus Bis(4-hydroxyphenyl)-diphenylethylen (Isomerengemisch), Hydrochinon, 2,6-Dihydroxynaphthalin, 4,4′-Dichlordiphenylsulfon und 2,6-Difluorbenzonitril.
In analoger Weise wie in Beispiel 8 wird ein Polymer aus 0,0251 mol Bis(4-hydroxyphenyl)-diphenylethylen, 0,0127 mol Hydrochinon und 0,0126 mol 2,6-Dihydroxynaphthalin, 0,0400 mol 4,4′-Dichlordiphenylsulfon, 0,0101 mol 2,6-Difluorbenzonitril und 0,0528 mol Kaliumcarbonat (Reaktionsbedingungen: 1h/225°C, 1h/248°C und 2h 45 Min./282°C) synthetisiert und isoliert. Das resultierende Polyethersulfon mit einer reduzierten Viskosität von 1,06 dl/g und einer Glasübergangstemperatur von 216°C ist in Methylenchlorid, in 1,3-Dioxolan, in Cyclohexanon und in N-Methylpyrrolidon zu mehr als 20 Gew.% löslich.

Beispiel 12:

Polyethersulfon aus Bis(4-hydroxyphenyl)-diphenylethylen (Isomerengemisch), 4,4′-Dihydroxybenzophenon, 4,4′-Dihydroxybiphenyl, Bisphenol A und 4,4′-Dichlordiphenylsulfon.
In analoger Weise wie in Beispiel 8 wird ein Polymer aus 0,0100 mol Bis(4-hydroxyphenyl)-diphenylethylen, 0,0101 mol 4,4′-Dihydroxybenzophenon, 0,0201 mol 4,4′-Dihydroxybiphenyl, 0,0101 mol Bisphenol A, 0,0500 mol 4,4′-Dichlordiphenylsulfon und 0,0526 mol Kaliumcarbonat (Reaktionsbedingungen: 1h/224°C, 1h/251°C und 3h/283 bis 285°C) synthetisiert und isoliert. Das resultierende Polyethersulfon mit einer reduzierten Viskosität von 0,23 dl/g und einer Glasübergangstemperatur von 170°C ist in Methylenchlorid, in 1,3-Dioxolan, in Cyclohexanon und in N-Methylpyrrolidon zu mehr als 20 Gew.% löslich.

Beispiel 13:

Polyethersulfon aus Bis(4-hydroxyphenyl)-diphenylethylen (1:1 Isomerengemisch aus basischer Etherspaltung) und 4,4-Dichlodiphenylsulfon.
In analoger Weise wie in Beispiel 8 wird ein Polymer aus 0,0100 mol Bis(4-hydroxyphenyl)-diphenylethylen und 0,0100 mol 4,4′-Dichlordiphenylsulfon und 0,010 mol Kaliumcarbonat (Reaktionsbedingungen: 1h/226°c, 1h/248°C und 4h/281°C) synthetisiert und isoliert. Das resultierende Polyethersulfon mit einer reduzierten Viskosität von 0,24 dl/g und einer Glasübergangstemperatur von 201°C ist in Methylenchlorid, in 1,3-Dioxolan, in Cyclohexanon und in N-Methylpyrrolidon zu mehr als 20 Gew.% löslich.

## Patentansprüche

1. Polyarylenether, die, bezogen auf die Gesamtmenge der im Polymer vorhandenen Strukturelemente 2-100 Mol% eines oder mehrerer wiederkehrender Strukturelemente der Formeln Ia, Ib oder Ic

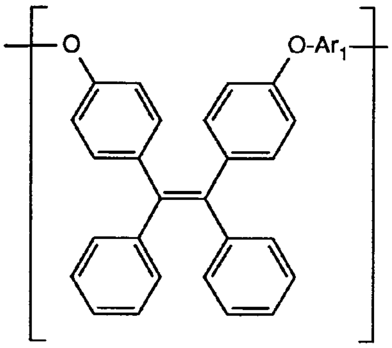 (Ia), 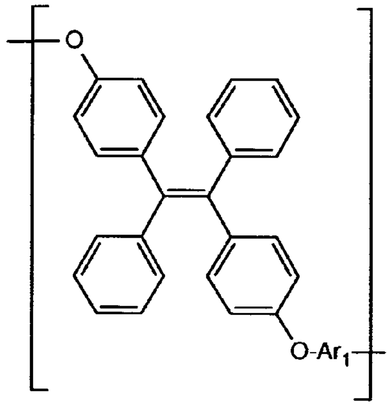 (Ib),

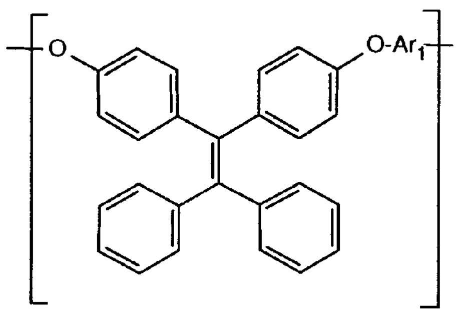 (Ic)

und 0-98 Mol% eines wiederkehrenden Strukturelementes der Formel II

$$-\!\!\left[-O-Ar_2-O-Ar_1-\right]\!\!- \qquad \text{(II)}$$

enthalten, worin $Ar_2$ einen unsubstituierten oder durch eine oder mehrere $C_1$-$C_4$-Alkylgruppen, $C_1$-$C_4$-Alkoxygruppen oder Halogenatome substituierten Rest der Formeln IIIa-IIIg bedeutet

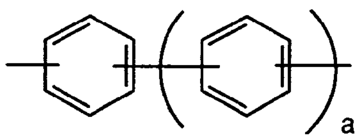 (IIIa), 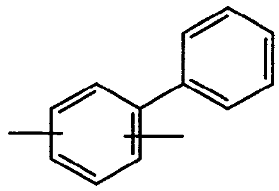 (IIIb),

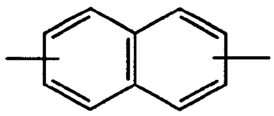 (IIIc), 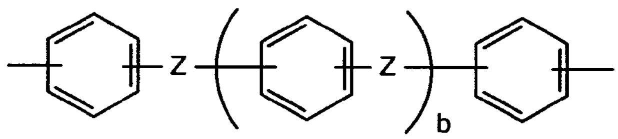 (IIId),

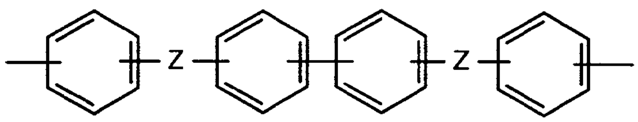 (IIIe),

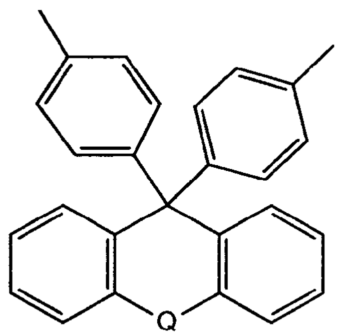 , 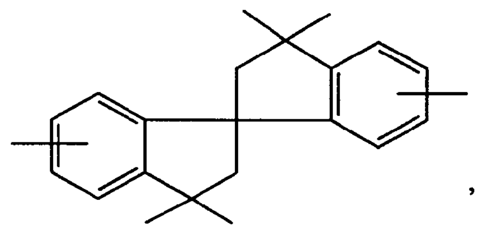 ,

(IIIg)

(IIIf)

wobei a und b für Null oder 1 und Z für -CO-, -SO2-, -SO-, -S-, -O-, $-C(CH_3)_2-$, $-C(CF_3)_2-$, $-CH_2-$ oder -$C(CH_3)(C_6H_5)$- stehen und Q für $-CH_2-$, -O-, -C(=O)- oder eine direkte Bindung steht, und $Ar_1$ einen unsubstituierten oder durch eine oder mehrere $C_1$-$C_4$-Alkylgruppen, $C_1$-$C_4$-Alkoxygruppen oder Halogenatome substituierten Rest der Formeln IVa, IVb oder IVc bedeutet

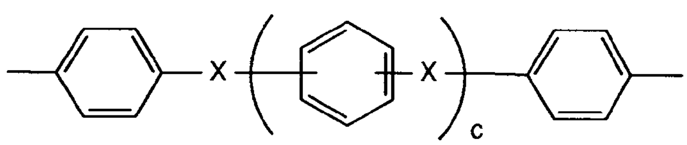 (IVa),

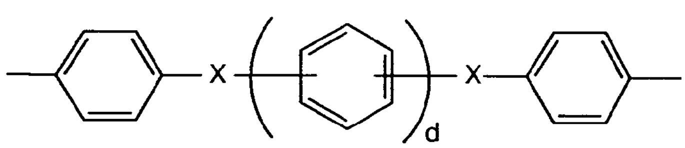 (IVb),

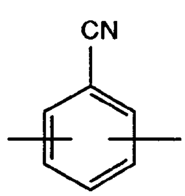 (IVc),

wobei c für Null, 1 oder 2 und d für 2 oder 3 stehen und X -CO-, $-SO_2-$ oder -SO- darstellt.

2. Polyarylenether nach Anspruch 1 mit einer reduzierten Viskosität von 0, 1 bis 2,0 dl/g, vorzugsweise von 0,3 bis 1,5 dl/g, gemessen bei 25°C an einer 1 Gew.%-igen Lösung in N-Methylpyrrolidon.

3. Polyarylenether nach Anspruch 1 oder 2, welche 5-100, vorzugsweise 10-100 Mol% eines wiederkehrenden Strukturelementes der Formel I und 0-95, vorzugsweise 0-90 Mol% eines wiederkehrenden Strukturelementes der Formel II enthalten.

4. Polyarylenether nach Anspruch 3, welche 25-100 Mol% eines wiederkehrenden Strukturelementes der Formel I und 0-75 Mol% eines wiederkehrenden Strukturelementes der Formel II enthalten.

5. Polyarylenether nach einem der Ansprüche 1 bis 4, worin das wiederkehrende Strukturelement der Formel I von einem Bis(4-hydroxyphenyl)-diphenylethylen-Isomerengemisch abgeleitet ist.

6. Polyarylenether nach Anspruch 1 enthaltend unsubstituierte Gruppen $Ar_1$ und $Ar_2$.

7. Polyarylenether nach Anspruch 1, worin X im Rest der Formeln IVa oder IVb $-SO_2-$ darstellt, insbesondere Polyarylenether, worin $Ar_1$ für den Rest

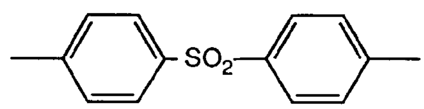

steht.

8. Polyarylenether nach Anspruch 1, worin $Ar_2$ für einen Rest der Formeln

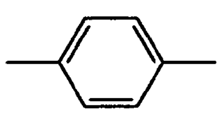 , 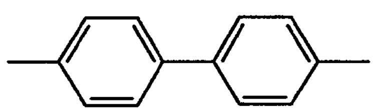 , 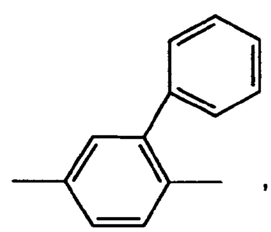 ,

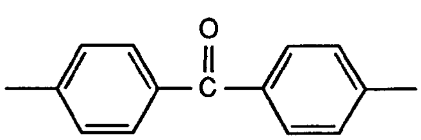 , 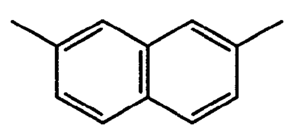 ,

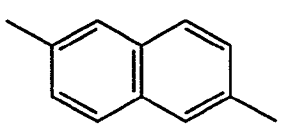 , 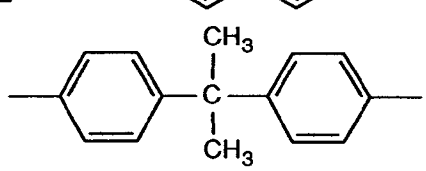

oder insbesondere

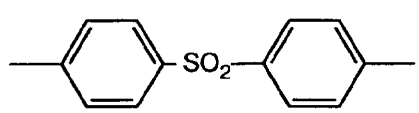

steht.

**9.** Polyarylenether gemäss Anspruch 3, worin in den Formeln I bzw. II $Ar_1$ und $Ar_2$ einen Rest der Formel

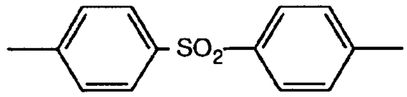

bedeuten, oder $Ar_1$ ein Rest der Formel

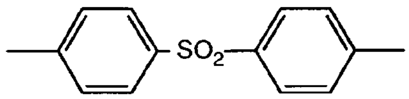

und $Ar_2$ ein Rest der Formel

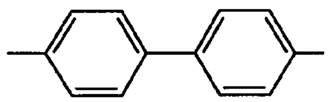

sind.

**10.** Verfahren zur Herstellung von Polyarylenethern gemäss Anspruch 1, wonach man ein oder mehrere Diphenole der Formeln Va, Vb oder Vc

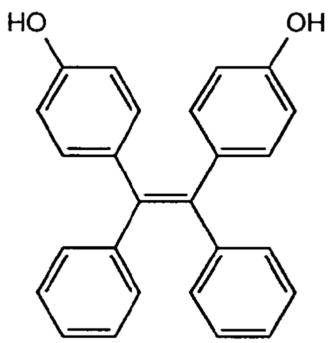

(Va),

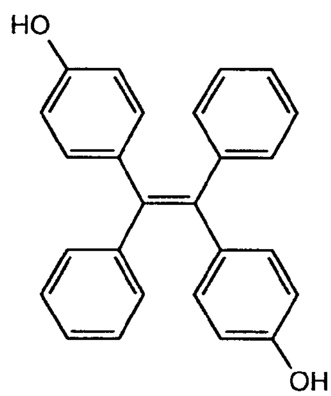

(Vb),

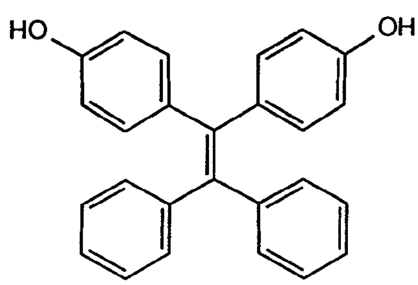

(Vc)

und gegebenenfalls ein Diphenol der Formel VI

$$HO\text{-}Ar_2\text{-}OH \quad (VI)$$

mit einem Dihalogenderivat der Formel VII

$$Hal\text{-}Ar_1\text{-}Hal \quad (VII)$$

in Gegenwart alkalischer Katalysatoren in einem polaren aprotischen Lösungsmittel polykondensiert, wobei $Ar_1$ und $Ar_2$ die in Anspruch 1 angegebene Bedeutung haben, und Hal für Halogen, insbesondere Fluor oder Chlor, steht, und wobei die relativen Mengen des Diphenols gemäss Formeln Va bis Vc und des Diphenols der Formel VI so gewählt werden, dass das resultierende Copolymer die im Anspruch 1

definierten Mengen der Strukturelemente der Formel I und II aufweist.

**11.** Verfahren gemäss Anspruch 10, wonach anstelle der Diphenole der Formel V oder VI die entsprechenden Alkalimetall- oder Erdalkalimetallphenolate eingesetzt werden.

**12.** Formkörper, Folien, Beschichtungen oder Klebeverbindungen enthaltend einen Polyarylenether nach Anspruch 1.

**13.** Faserverbund enthaltend Verstärkungsfasern und als Matrixharz Polyarylenether nach Anspruch 1.

**14.** Lösung enthaltend 1 bis 75 Gew.% eines Polyarylenethers gemäss Anspruch 1, gelöst in einem organischen Lösungsmittel.

**15.** Isomerengemisch enthaltend die Diphenole der Formeln Va, Vb und Vc nach Anspruch 10, erhältlich durch Umsetzung von 4-Methoxybenzophenon mit $PCl_5$ und anschliessende Dimerisierung des entstandenen Dichlormethanderivats

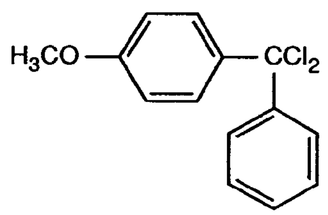

mit elementarem Kupfer zum Dimethoxytetraphenylethylen, dadurch gekennzeichnet, dass die Methyletherspaltung in stark saurem Medium vorgenommen wird.

Europäisches Patentamt

# EUROPÄISCHER RECHERCHENBERICHT

Nummer der Anmeldung

EP 91 81 0558

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | US-A-2 571 954 (R. S. SHELTON ET AL.)<br>--- | | C08G65/40 |
| A | CH-A-431 495 (AB FERROSAN)<br>----- | | |
| | | | **RECHERCHIERTE SACHGEBIETE (Int. Cl.5 )** |
| | | | C08G<br>C07C |

Der vorliegende Recherchenbericht wurde für alle Patentansprüche erstellt

| Recherchenort | Abschlußdatum der Recherche | Prüfer |
|---|---|---|
| DEN HAAG | 08 NOVEMBER 1991 | PRAS JL |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.......................................................................................................
& : Mitglied der gleichen Patentfamilie, übereinstimmendes Dokument

EPO FORM 1503 03.82 (P0403)